# EUROPEAN PATENT SPECIFICATION

(11) **EP 4 161 377 B1**
(45) Date of publication and mention of the grant of the patent: **21.08.2024**
(21) Application number: 21733073.7
(22) Date of filing: 08.06.2021
(51) Int. Cl.: A61B 5/0285, A61B 5/0535, A61B 5/341, A61B 5/333

(54) **METHOD AND DEVICE FOR MULTIDIMENSIONAL ANALYSIS OF THE DYNAMICS OF CARDIAC ACTIVITY**
VERFAHREN UND VORRICHTUNG ZUR MEHRDIMENSIONALEN ANALYSE DER DYNAMIK DER HERZAKTIVITÄT
PROCÉDÉ ET DISPOSITIF D'ANALYSE MULTIDIMENSIONNELLE DE LA DYNAMIQUE DE L'ACTIVITÉ CARDIAQUE

(30) Priority: 08.06.2020 EP 20178686; 08.06.2020 PL 43423320
(43) Date of publication of application: 12.04.2023
(73) Proprietor: EFM Sp. z o.o., 00-511 Warszawa (PL)
(72) Inventor: JAMROZY, Milosz, 05-084 Marianow (PL); LEYKO, Tomasz, 02-803 Warszawa (PL); CUDNY, Aleksander, 02-677 Warszawa (PL)
(74) Representative: AOMB Polska Sp. z.o.o.
(86) International application number: PCT/EP2021/065371
(87) International publication number: WO 2021/250048

(56) References cited:
- CN-Y- 2 178 501
- DE-B1- 2 620 285
- US-A- 4 106 495
- US-A1- 2009 262 109
- PARASTOO DEHKORDI ET AL: "Comparison of Different Methods for Estimating Cardiac Timings: A Comprehensive Multimodal Echocardiography Investigation", FRONTIERS IN PHYSIOLOGY, vol. 10, 22 August 2019 (2019-08-22), XP055770838, DOI: 10.3389/fphys.2019.01057
- HANNE O. AUSTAD ET AL: "An Unobtrusive Wearable Device for Ambulatory Monitoring of Pulse Transit Time to Estimate Central Blood Pressure :", PROCEEDINGS OF THE 9TH INTERNATIONAL JOINT CONFERENCE ON BIOMEDICAL ENGINEERING SYSTEMS AND TECHNOLOGIES, 23 February 2016 (2016-02-23), pages 179 - 186, XP055639045, ISBN: 978-989-7581-70-0, DOI: 10.5220/0005701401790186

## Description

The invention relates to a method for measuring energy of cardiac activity and time course of cardiac contraction by measuring impedances and voltages, and a device for implementing this method.

There are known devices for monitoring various parameters of cardiac activity including for instance blood pressure, heart rate (HR) using ECG, stroke volume (minute volume) using cardiac impedance, or haemoglobin oxygen saturation (SpOz) using pulse oximeter. These are not, however, devices which are dedicated to searching for dependencies between the time course of cardiac contraction and the stimulation and result of the contraction. The contraction time course can be observed using methods such as: echocardiography, magnetic resonance, computer tomography, nuclear medicine methods. Each of them requires an expensive equipment, trained personnel and experience. There is currently no known device on the market which is used for searching a relationship between the aforementioned phenomena.

There is also no known device that would simultaneously perform vectorcardiography and three-dimensional cardiac impedance. A 3D-rheometry measurement with classical electrocardiography is used in systems for navigation during heart ablation procedures in arrhythmia treatment. In this case, the real and imaginary parts of the impedance are not measured - what is only measured is the resistance, and additionally, the measurement is performed between a tool in the human body and the electrodes stuck to the body. The cardiac activity related changes in chest impedance represent a disturbance in this measurement, an expected information is the position of the tool with respect to the measuring electrodes - that's why a heart rate synchronous averaging is used to stabilize the reconstructed images.

US20110295127A discloses a device and method for vibro-acoustic detection of cardiac arrhythmia. Measurements of vibrations are performed and based on the frequency and amplitude changes it is possible to determine if and which disturbances occur. The dynamics of cardiac activity is nonlinear because of many independent systems of cardiac rhythm regulation. For that reason, a frequency analysis, which requires linearity, does not fall within the scope of the disclosed invention.

US20150374256A1 discloses a device which can measure the dynamics of cardiac activity and examine the flow of fluids. The measurement is performed by using leads on the patient's body, where also current and voltage, pressure and vibrations are probed. The dynamics which are determined by the disclosed device relate to blood - the detected parameters are hemodynamic parameters, and the determination thereof consists in applying a model allowing for estimation of the blood motion and in determining, for instance, the cardiac stroke volume. The solution disclosed herein is not limited to the assessment of hemodynamics.

US2009/262109 discloses a system and a method of mapping the patient body in order to navigate the device inserted inside the patient's body. Among other elements, the system includes means For measuring of impedance in three orthogonal axes (with different current frequencies on each axe) as well as separate means for ordinary ECG measurements using three, electrodes separate front the ones used in case impedance measurements. The device inserted in the patient's body is a catheter having a balloon for measuring blood pressure and reference electrode used to estimate the position of the catheter based on measured impedance values.

Publication of Prastoo D. et. al. (Comparison of Different Methods for Estimating Cardiac Timings: A comprehensive Multimodal Echocardiography Investigation, Frontiers in Physiology, vol. 10, 22.08.2019) discloses a method involving simultaneous ECG, ICG and PCG measurements. However, no information is disclosed relating to three-dimensional measurements or using electrodes located on three orthogonal axes.

CN2178501 discloses a impedance measurement method using three orthogonal axes. However CN2178501 is silent on using various current frequencies on each exe as well as on combining such measurement with three dimensional ECG or tissue movement measurement.

The solution disclosed herein allows for preliminary assessment of cardiac activity and qualification For an expensive image diagnostics based on a 3D-measurement of ECG signal, 3D-measurement of impedance rheometry, and, preferably, acoustic, vibrational or ultrasonic signals.

The measurement apparatus described below will enable an advanced analysis of signals to allow differentiation of heart diseases by evaluation of the mode of heart motion in the chest. It will allow for non-invasive diagnostics of various heart diseases, which can additionally be automated and available in every clinic.

The invention relates to a method for measuring multidimensional dynamics of cardiac activity comprising simultaneous measurement of three dimensional ECG and three dimensional impedance rheometry, wherein the impedance rheometry measurement comprises simultaneous generation of applied current of different frequencies for three orthogonal axes and an impedance measurement for the same or other three orthogonal axes and wherein the impedance rheometry measurement and the ECG measurement are carried out for the same three orthogonal axes.

The method comprises a tissue motion measurement, simultaneous with the ECG measurement and the impedance rheometry measurement. The measurement applies in particular to tissues that move in the chest, such as contractile tissues (muscles) and those that are moved by the contractile tissues (e.g. lung tissue).

The tissue motion measurement is performed with a sensor placed outside the patient body selected from among sensors including: membrane, auscultation funnel, accelerometer, microphone, piezoelectric sensor.

According to the present invention, the term "outside the patient body" used with respect to tissue motion measurements means that the tissue measurement sensor is used in substantially non-invasive manner, preferably the sensor is placed on the patient's skin.

Preferably, the impedance rheometry measurement is performed for three orthogonal axes whose intersection is in the heart area.

Preferably, the ECG measurement and/or the impedance rheometry measurement and/or the generation of applied current are carried out using electrodes in Frank configuration or in Leyko-Jamrozy configuration, where
the Z axis is determined by the first electrode located in the right supraclavicular Fossa and the second electrode located in the position of the apex beat,
the X axis is determined by the third electrode located at Erb's point (the third or fourth intercostal space, at the left edge of the sternum) and the fourth electrode in the right posterior axillary line at its intersection with the line perpendicular to the Z axis and passing through the first electrode of the X axis,
the Y axis is determined by the fifth electrode located in the left posterior axillary line at the height of the third or fourth intercostal space and the sixth electrode on the surface of the body at the exit point of the line passing through the first point of the Y axis and the point intersecting the X and Z axes.

Preferably, the measurements are carried out using one time base.

Preferably, the measurement step is followed by a step of calculating currents, energy and work related to myocardium activity based on the measurement data.

Preferably, the method further comprises a step of assessing the dynamics of changes in the signal waveforms.

The invention relates also to a device for multidimensional analysis of the dynamics of cardiac activity adapted for simultaneous ECG and impedance rheometry measurement, comprising
a three dimensional ECG measurement system,
a three dimensional impedance rheometry measurement system, a system for generation of applied currents,
an electrode system comprising electrodes arranged on three orthogonal axes, comprising separate electrodes of the ECG measurement system, the impedance rheometry measurement system and the system for generation of applied currents,
wherein the ECG measurement system, the impedance rheometry measurement system and the system for generation of applied currents are adapted for simultaneous measurement in three orthogonal axes, and further the frequency of the applied current is different for each of the three orthogonal axes, and
at least one tissue motion sensor adapted to be disposed outside of the patient body, selected from among sensors including: membrane, auscultation funnel, accelerometer, microphone, piezoelectric sensor.

Preferably, all systems of the device have a common time base.

Preferably, the device comprises an interface for data transfer.

Preferably, the device comprises a pulse oximetry device with PTT measurement.

The invention will be now explained in detail, in an embodiment, with reference to accompanying Figures, wherein:
- Fig. 1: shows schematically components of the device according to the invention,
- Fig. 2: shows the Frank lead configuration,
- Fig. 3: shows the data obtained from measurements in a healthy subject,
- Fig. 4: shows the data obtained from measurements in a patient with cardiac disorders.

The device is part of a system that receives signals and analyzes them. The task of the system is to extract information on generally understood dynamics of cardiac activity - not limited to hemodynamics (blood motion in the heart and vessels), but covering the motion of cardiac tissues in the chest, assessment of energy generated by the heart through estimation from current and impedance measurements. The task of the system is to obtain information on the phenomena related to the electrical aspect of cardiac activity in three-dimensional terms (ECG and 3D-cardioimpedance), to obtain holistic information about the motion of cardiac tissues (microphone, accelerometer, ultrasonic measurement), and possibly also about the condition of vessels, the vascular resistance and the pulse wave (as a PTT measurement). Collecting this information synchronously allows for analyzing the relationships between individual contractions and studying the dynamics of various processes. Due to the presence of several regulation systems of (heart rate) stimulation, the cardiac activity is non-linear and the stability of the activity observed in healthy condition, when measured at rest, disappears in sick patients.

Along with the disease and the resulting chaotic activity of the overloaded heart, we observe a very rich and complex dynamics of motion of cardiac tissues, a decrease in the efficiency of heart as a pump and an increase in the energy input needed to maintain the required flows in the body. All these aspects can be measured with the solution according to the present invention.

The device for multidimensional analysis of the dynamics of cardiac activity according to the present invention comprises leads for the three dimensional ECG measurement and leads for three dimensional impedance rheometry in three orthogonal axes. The leads are attached to the body with electrodes. The lead system used for impedance rheometry has preferably a centre in the heart area. The device comprises three electrode signal measuring systems, three systems for generation of applied currents and at least one tissue motion sensor. Preferably, the device comprises a communication interface with the user. The signal receiving systems and the systems for generation of applied currents are configured for simultaneous measurement in three orthogonal axes. The frequency of the applied current is different for each of the three orthogonal axes.

Correct data collection must meet the following conditions:
- The impedance measurement must be carried out for three orthogonal axes for three different frequencies. The measurement cannot be performed sequentially, and further the electrode system may be different from the lead system (only the orthogonality of the axes is important).
- The impedance and voltage measurements need not be carried out in the same axes.
- All measurements must be made simultaneously, sequential measurements for subsequent axes are not allowed.

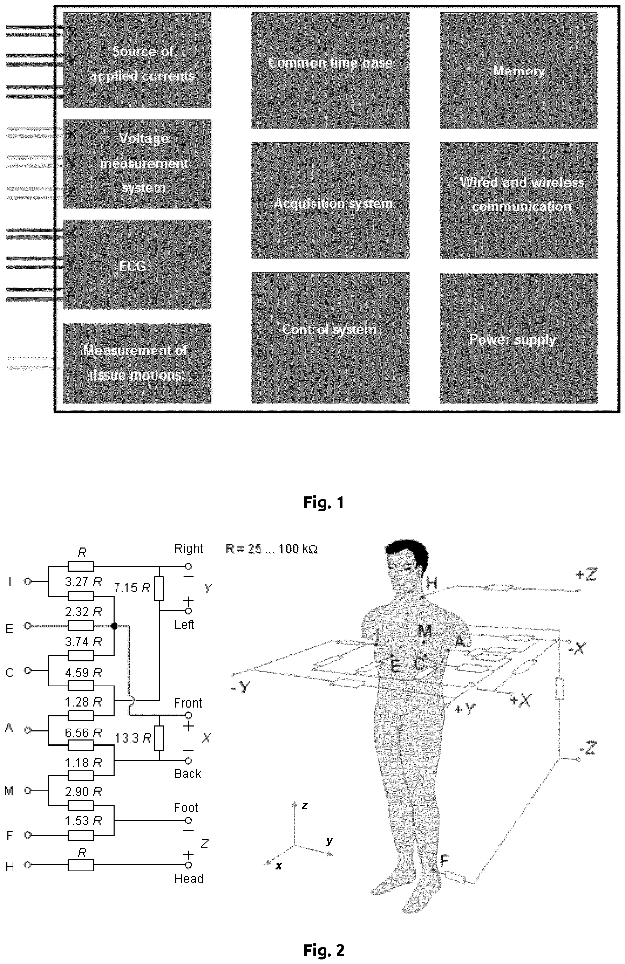

The real and imaginary parts of impedance are measured simultaneously in each axis, where the ECG is measured. As shown in Fig. 2, the measurement is carried out in three orthogonal axes: the X axis (measurement points E and M), the Y axis (measurement points I and A), the Z axis (measurement points F and H). Due to this measurement, it will be possible to use information about the tissue resistance and voltage changes in perpendicular axes. This enables estimation of currents, energy and work related to myocardium activity.

Functionally, the device according to the embodiment can be divided into several modules, i.e. measuring, communication, control, memory, power supply, information display and user communication module, as shown in Fig. 1. It should be emphasized that the information display and user communication module or the communication module are optional. Small, portable devices will be mainly used to record and save data, while in the case of testing it may turn out that further data transmission is not necessary.

The device is designed to perform synchronously (preferably using one time base) measurement of 3 orthogonal ECG leads, 3 orthogonal reactance and resistance measurements, and at least one measurement related to the tissue motion in chest, and preferably other additional signals are also measured. Such a measurement of tissue motion may be, for instance, a measurement of an acoustic signal with a diaphragm or auscultation funnel, a vibration signal (measured by any method), a Doppler signal measured by pulse or continuous wave method, with one or multiple transducers.

An important difference between the measurements with the present device and with devices enabling measurements of similar signals, is that the present device does not use models enabling reference to physiology or anatomy (e.g., by setting up a model allowing for estimation of the stroke volume, tissue hydration from a rheometric signal, or mapping information on a reconstructed anatomical model - a tissue Doppler). The essence of the recording provided by the present device is that the measured physical properties are simultaneously recorded using transducers - for instance voltage in orthogonal leads expressed in mV, resistance expressed in ohms, pressure changes in the auscultation funnel expressed, for instance, in pascals, etc. Therefore, the arrangement of electrodes differs from typical applications, which due to the models behind them allows For diagnosing - for instance, the use of Wilson leads in ECG enables the assessment of left ventricular hypertrophy based on meeting the criteria For voltages in individual channels.

Due to the fact that the applied currents in each of the three orthogonal axes will flow simultaneously, different frequencies must be used for each channel. This means that the measured signal in each of the three receiving systems will represent the superposition of three frequencies. To perform a high quality measurement, special signal processing path and synchronization must be used which prevents the use of 3 independent rheometers even if they can be tuned to different frequencies.

The collected signals are subsequently used for analysis with the aim to assess the dynamics of cardiac activity (not to be confused with the hemodynamic assessment, which describes the dynamics of the blood flow). To this end, the whole set of collected signals is assessed, including electrical (changes of voltage and impedance in chest) and mechanical (tissue motion) phenomena. Together, they will set up a multidimensional picture of phenomena over time, the variability of which is studied and evaluated.

The device preferably uses an electrode system that forms a Frank lead system or a Leyko-Jamrozy lead system, where the Z axis is determined by the first electrode located in the right supraclavicular Fossa and the second electrode located in the position of the apex beat, the X axis is determined by the third electrode located at Erb's point (the third or fourth intercostal space, at the left edge of the sternum) and the fourth electrode in the right posterior axillary line at its intersection with the line perpendicular to the Z axis and passing through the first electrode of the X axis, the Y axis is determined by the fifth electrode located in the left posterior axillary line at the height of the third or fourth intercostal space and the sixth electrode on the surface of the body at the exit point of the line passing through the first point of the Y axis and the point intersecting the X and Z axes.

The motion sensor should be located in the subsequent intercostal space above the position of the apex beat.

The set of three electrodes is placed on one triple adhesive tape. The centre of the orthogonal coordinate system in the heart area.

Preferably, the device has a lead system for ECG measurement and a lead system for impedance rheometry, which systems have their centres essentially in the same place, and even more preferably the lead systems are the same for ECG and impedance rheometry measurements. In the most favourable variant, the impedance and voltage measurements are performed in the same axes.

Preferably, the leads are connected to the body through triple electrodes.

The measuring system in the rheometry measurement and the system For generation of applied currents can use separate or the same electrodes.

Preferably, all device systems have a common time base. It is possible to synchronize systems without a common time base, an easier and more practical synchronization can be, however, achieved through a common time base.

The method of measuring multidimensional dynamics of cardiac activity according to the invention comprises steps of attaching the lead electrodes to the body, simultaneously measuring ECG and impedance rheometry in three orthogonal axes by simultaneously generating applied current with different frequencies for each axis and measuring voltages between electrodes and calculating currents, energy and work related to myocardial activity based on the measurement data. Preferably, the device or the data analyzing system uses the data to assess the dynamics of signal waveform changes.

The device can also be equipped with an accelerometer and/or pulse oximetry device with PTT (Pulse Transit Time) measurement.

The device may also have a module for multi-channel measurement of tissue motion with continuous wave or pulse Doppler method (similar to the method used in CTG or tissue Doppler in echocardiography).

The device may be equipped with an additional accelerometer for measuring the patient's physical activity.

The device can be implemented in a stationary or portable form. It can be integrated into one device with other peripherals (screens, touch screens, keyboards, mice, glasses for virtual reality, etc., on a mobile supporting structure) or consist of components connected in various ways (e.g., the main unit and cards/extensions/components connected to it to expand functionality). The connection can be wired or wireless. The measuring device can also be of the Holter type - for signal recording lasting many hours, or stationary bedside.

Both stationary and portable devices can be battery powered, using disposable batteries, rechargeable batteries or other replaceable power supply sources. A stationary device can be operated using mains power supply. The devices can be equipped with wired interfaces for data uploading and downloading to and from devices (e.g. USB, SD cards, MMC memories, RJ45 network input) as well as with wireless ones (e.g., WiFi, Bluetooth, cellular modems). Both stationary and portable devices may have sockets for connecting test leads. The device allows for integration of wireless measuring devices instead of wired solutions.

Measuring impedance in an orthogonal system shows how resistance and reactance change over time during a cardiac contraction. When the heart is healthy, each beat in a rest measurement is similar to the previous one, and the time course of changes in the above parameters is cyclical. In the case of the heart affected by dilated heart disease, the tissue motion is reduced, which can result in reduced reactance and impedance changes. In left ventricular hypertrophy, changes in reactance and impedance can be more strongly expressed. An ECG measurement with the three conditions described above may indicate that the differences in recording may be difficult to notice. An ECG measurement in an orthogonal system, associated with an orthogonal impedance measurement system, allows for linking the stimulation in the form of voltage measured in the ECG to the change in tissue resistance in each of the measured directions. This is unique information because it shows the energy trajectory in a three-dimensional system, which is not available by any measurement method used so far. The excitation observed in the ECG is a control stimulation leading to contraction. A myocardial contraction results in a change of the resistance and reactance in the chest and has a hemodynamic effect. Until now, only the hemodynamic effect was observed, with no attention paid to what happens to resistance and reactance, and these quantities carry information about the time course of the contraction. Inefficient myocardium is not able to produce a significant hemodynamic effect (the essence of the failure is a low minute stroke volume), and still can produce a significant effect related to a change in chest resistance (strong motion of a pathologically hypertrophied myocardium). Supplementing the information by adding other sensors allowing for observation of the effects of cardiac contractions - including acoustic, vibration, ultrasonic measurement of signals from the heart moving in the chest, enables a more complete observation of the occurring phenomena and analysis of the dynamics of cardiac activity.

### Example of measurements for healthy and sick patients

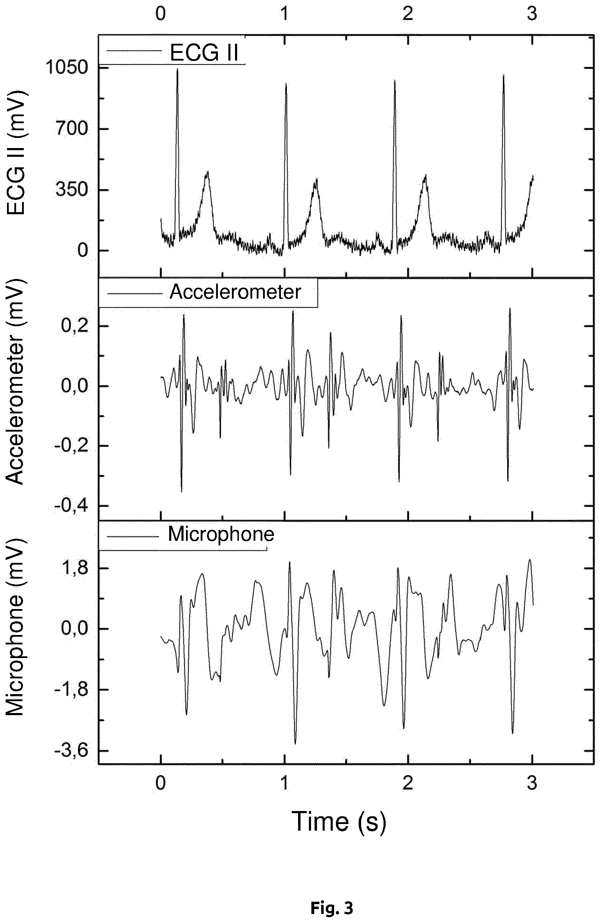

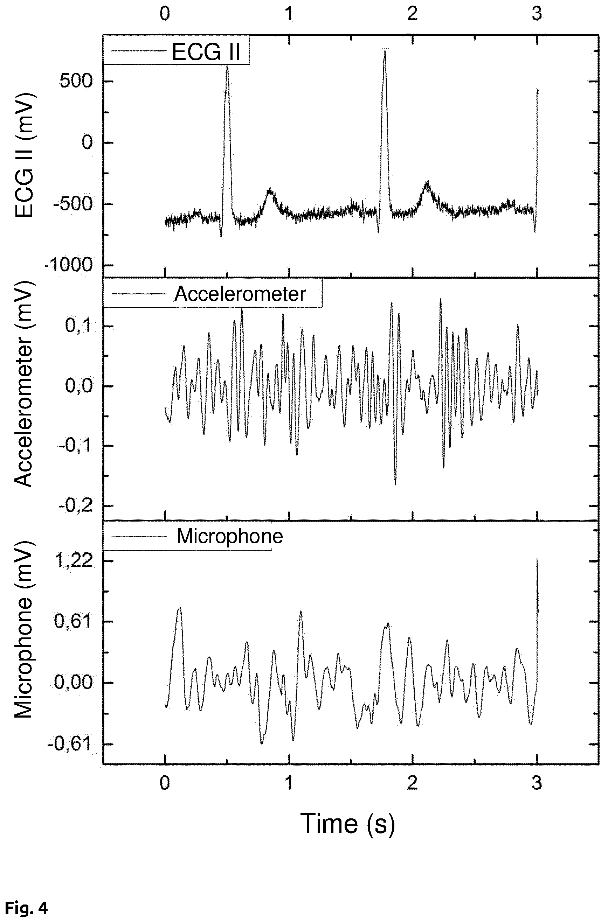

The patient had an ECG and a stethoscope and accelerometer were placed on the surface of the chest. The measurement was synchronous. In healthy subject, each heartbeat (observed on ECG) generated in the microphone and the accelerometer a signal waveform with a certain pattern (Fig. 3). The pattern was not observed in the sick person - the waveform seemed unrelated to the ECG signal (Fig. 4).

The analysis of the signal from the microphone collected during a 30 second measurement using analytical methods allowed to distinguish between the healthy and sick patients with 80% efficiency.

## Claims

1. Method for measuring multidimensional dynamics of cardiac activity comprising simultaneous measurement of:
three dimensional ECG,
three dimensional impedance rheometry,
tissue motion, wherein the tissue motion measurement is performed with a sensor placed outside the patient body selected from among sensors including: membrane, auscultation Funnel, accelerometer, microphone, piezoelectric sensor,
wherein the impedance rheometry measurement comprises simultaneous generation of applied current of different frequencies for three orthogonal axes and an impedance measurement for the same or other three orthogonal axes, and
wherein the impedance rheometry measurement and the ECG measurement are carried out for the same three orthogonal axes.

2. Method according to claim 1, **characterised in that** the impedance rheometry measurement is performed for three orthogonal axes whose intersection is in the heart area.

3. Method according to any of the foregoing claims, **characterised in that** the ECG measurement and/or the impedance rheometry measurement and/or the generation of applied current are carried out using the electrodes in Frank configuration or in Leyko-Jamrozy configuration, where
the Z axis is determined by the first electrode located in the right supraclavicular Fossa and the second electrode located in the position of the apex beat,
the X axis is determined by the third electrode located at Erb's point (the third or fourth intercostal space, at the left edge of the sternum) and the fourth electrode in the right posterior axillary line at its intersection with the line perpendicular to the Z axis and passing through the first electrode of the X axis,
the Y axis is determined by the fifth electrode located in the left posterior axillary line at the height of the third or fourth intercostal space and the sixth electrode on the surface of the body at the exit point of the line passing through the first point of the Y axis and the point intersecting the X and Z axes.

4. Method according to any of the foregoing claims, **characterised in that** the measurements are carried out using one time base.

5. Method according to any of the foregoing claims, **characterised in that** the measurement step is followed by a step of calculating currents, energy and work related to myocardium activity based on the measurement data.

6. Method according to any of the foregoing claims, **characterised in that** it further comprises a step of assessing the dynamics of changes in the signal waveforms.

7. Device for multidimensional analysis of the dynamics of cardiac activity adapted for simultaneous ECG and impedance rheometry measurement, comprising:
a three dimensional ECG measurement system,
a three dimensional impedance rheometry measurement system and a system for generation of applied currents,
an electrode system comprising electrodes arranged on three orthogonal axes, comprising separate electrodes of the ECG measurement system, the impedance rheometry measurement system and the system for generation of applied currents,
wherein the ECG measurement system, the impedance rheometry measurement system and the system for generation of applied currents are adapted for simultaneous measurement in three orthogonal axes, and further the frequency of the applied current is different for each of the three orthogonal axes, and
at least one tissue motion sensor adapted to be disposed outside of the patient body, selected from among sensors including: membrane, auscultation funnel, accelerometer, microphone, piezoelectric sensor.

8. Device according to claim 7, **characterised in that** all systems of the device have a common time base.

9. Device according to claim 7 or 8, **characterised in that** the device comprises an interface for data transfer.

10. Device according to any of preceding claim 7-9 **characterised in that** the device comprises a pulse oximetry device with PTT measurement.

## Patentansprüche

1. Verfahren zur Messung der multidimensionalen Dynamik der Herzaktivität mit gleichzeitiger Messung von:
dreidimensionalem EKG,
dreidimensionaler Impedanzrheometrie,
Gewebebewegung, wobei die Messung der Gewebebewegung mit einem Sensor durchgeführt wird, der außerhalb des Patientenkörpers angeordnet ist und aus folgenden Sensoren ausgewählt ist: Membran, Auskultationstrichter, Beschleunigungsmesser, Mikrofon, piezoelektrischer Sensor,
wobei die Rheo-Impedanzmessung die gleichzeitige Erzeugung eines angelegten Stroms mit unterschiedlichen Frequenzen für drei orthogonale Achsen und eine Impedanzmessung für die gleichen oder andere drei orthogonale Achsen umfasst, und
wobei die Rheo-Impedanzmessung und die EKG-Messung für die gleichen drei orthogonalen Achsen durchgeführt werden.

2. Verfahren nach Anspruch 1, **dadurch gekennzeichnet, dass** die Rheo-Impedanzmessung für drei orthogonale Achsen durchgeführt wird, deren Schnittpunkt im Herzbereich liegt.

3. Verfahren nach einem der vorhergehenden Ansprüche, **dadurch gekennzeichnet, dass** die EKG-Messung und/oder die Rheo-Impedanzmessung und/oder die Erzeugung von angelegtem Strom unter Verwendung der Elektroden in offener Konfiguration oder in Leyko-Jamrozy-Konfiguration durchgeführt wird, wobei
die Z-Achse durch die erste Elektrode, die sich in der rechten supraklavikulären Fossa befindet, und die zweite Elektrode, die sich in der Position des Apex-Schlags befindet, bestimmt wird,
die X-Achse durch die dritte Elektrode, die sich am Erb'schen Punkt (dem dritten oder vierten Zwischenrippenraum, am linken Rand des Brustbeins) und die vierte Elektrode in der rechten hinteren Axillarlinie an ihrem Schnittpunkt mit der Linie, die senkrecht zur Z-Achse verläuft und durch die erste Elektrode der X-Achse geht, bestimmt wird,
die Y-Achse wird durch die fünfte Elektrode bestimmt, die sich in der linken hinteren Axillarlinie in Höhe des dritten oder vierten Zwischenrippenraums befindet, und die sechste Elektrode auf der Körperoberfläche am Austrittspunkt der Linie, die durch den ersten Punkt der Y-Achse und den Schnittpunkt der X- und Z-Achse verläuft.

4. Verfahren nach einem der vorgenannten Ansprüche, **dadurch gekennzeichnet, dass** die Messungen mit einer Zeitbasis durchgeführt werden.

5. Verfahren nach einem der vorgenannten Ansprüche, **dadurch gekennzeichnet, dass** sich an den Messschritt ein Schritt der Berechnung von Strömen, Energie und Arbeit in Bezug auf die Herzmuskelaktivität auf der Grundlage der Messdaten anschließt.

6. Verfahren nach einem der vorgenannten Ansprüche, **dadurch gekennzeichnet, dass** es außerdem einen Schritt zur Bewertung der Dynamik von Veränderungen in den Signalverläufen umfasst.

7. Gerät zur multidimensionalen Analyse der Dynamik der Herzaktivität, die für die gleichzeitige Messung von EKG und Rheo-Impedanzmessung geeignet ist, umfassend:
ein dreidimensionales EKG-Messsystem,
ein dreidimensionales System zur Rheo-Impedanzmessung und ein System zur Erzeugung von angelegten Strömen,
ein Elektrodensystem, das auf drei orthogonalen Achsen angeordnete Elektroden umfasst, die separate Elektroden des EKG-Messsystems, des Rheo-Impedanz-Messsystems und des Systems zur Erzeugung von angelegten Strömen umfassen,
wobei das EKG-Messsystem, das Rheo-Impedanz-Messsystem und das System zur Erzeugung angelegter Ströme für die gleichzeitige Messung in drei orthogonalen Achsen angepasst sind und ferner die Frequenz des angelegten Stroms für jede der drei orthogonalen Achsen unterschiedlich ist, und
mindestens einen Gewebsbewegungssensor, der außerhalb des Patientenkörpers angeordnet werden kann, ausgewählt aus den folgenden Sensoren: Membran, Auskultationstrichter, Beschleunigungsmesser, Mikrofon, piezoelektrischer Sensor.

8. Gerät nach Anspruch 7, **dadurch gekennzeichnet, dass** alle Systeme des Geräts eine gemeinsame Zeitbasis haben.

9. Gerät nach Anspruch 7 oder 8, **dadurch gekennzeichnet, dass** das Gerät eine Schnittstelle zur Datenübertragung umfasst.

10. Gerät nach einem der vorhergehenden Ansprüche 7-9, **dadurch gekennzeichnet, dass** das Gerät ein Pulsoximetriegerät mit PTT-Messung umfasst.

## Revendications

1. Procédé de mesure de la dynamique multidimensionnelle de l'activité cardiaque comprenant la mesure simultanée de :
ECG tridimensionnel,
rhéométrie d'impédance tridimensionnelle,
mouvement des tissus, la mesure du mouvement des tissus étant effectuée à l'aide d'un capteur placé à l'extérieur du corps du patient, choisi parmi les capteurs comprenant : une membrane, un entonnoir d'auscultation, un accéléromètre, un microphone, un capteur piézoélectrique,
dans lequel la mesure de rhéométrie d'impédance comprend la génération simultanée d'un courant appliqué de différentes fréquences pour trois axes orthogonaux et une mesure d'impédance pour le même ou d'autres trois axes orthogonaux, et
dans lequel la mesure de rhéométrie d'impédance et la mesure ECG sont effectuées pour les trois mêmes axes orthogonaux.

2. Procédé selon la revendication 1, **caractérisé en ce que** la mesure de rhéométrie d'impédance est effectuée pour trois axes orthogonaux dont l'intersection se situe dans la zone cardiaque.

3. Procédé selon l'une quelconque des revendications précédentes, **caractérisé en ce que** la mesure ECG et/ou la mesure de rhéométrie d'impédance et/ou la génération de courant appliqué sont effectuées en utilisant les électrodes en configuration Frank ou en configuration Leyko-Jamrozy, où
l'axe Z est déterminé par la première électrode située dans la fosse supraclaviculaire droite et la seconde électrode située dans la position du battement d'apex,
l'axe X est déterminé par la troisième électrode située au point d'Erb (troisième ou quatrième espace intercostal, au bord gauche du sternum) et la quatrième électrode dans la ligne axillaire postérieure droite à son intersection avec la ligne perpendiculaire à l'axe Z et passant par la première électrode de l'axe X,
l'axe Y est déterminé par la cinquième électrode située dans la ligne axillaire postérieure gauche à la hauteur du troisième ou du quatrième espace intercostal et la sixième électrode sur
la surface du corps au point de sortie de la ligne passant par le premier point de l'axe Y et le point d'intersection des axes X et Z.

4. Procédé selon l'une quelconque des revendications précédentes, **caractérisé en ce que** les mesures sont effectuées à l'aide d'une base de temps.

5. Procédé selon l'une quelconque des revendications précédentes, **caractérisé en ce que** l'étape de mesure est suivie d'une étape de calcul des courants, de l'énergie et du travail liés à l'activité du myocarde sur la base des données de mesure.

6. Procédé selon l'une quelconque des revendications précédentes, **caractérisé en ce qu'**il comprend en outre une étape d'évaluation de la dynamique des changements dans les formes d'onde du signal.

7. Dispositif d'analyse multidimensionnelle de la dynamique de l'activité cardiaque adapté à la mesure simultanée de l'ECG et de la rhéométrie d'impédance, comprenant :
un système de mesure ECG tridimensionnel,
un système de mesure de rhéométrie d'impédance tridimensionnelle et un système de génération de courants appliqués,
un système d'électrodes comprenant des électrodes disposées sur trois axes orthogonaux, comprenant des électrodes séparées du système de mesure ECG, du système de mesure de rhéométrie d'impédance et du système de génération de courants appliqués,
dans lequel le système de mesure ECG, le système de mesure de rhéométrie d'impédance et le système de génération de courants appliqués sont adaptés à la mesure simultanée dans trois axes orthogonaux, et dans lequel la fréquence du courant appliqué est différente pour chacun des trois axes orthogonaux, et
au moins un capteur de mouvement des tissus adapté pour être placé à l'extérieur du corps du patient, choisi parmi les capteurs suivants : une membrane, un entonnoir d'auscultation, un accéléromètre, un microphone, un capteur piézoélectrique.

8. Dispositif selon la revendication 7, **caractérisé en ce que** tous les systèmes du dispositif ont une base de temps commune.

9. Dispositif selon la revendication 7 ou 8, **caractérisé en ce que** le dispositif comprend une interface pour le transfert de données.

10. Dispositif selon l'une quelconque des revendications 7 à 9 précédentes, **caractérisé en ce que** le dispositif comprend un dispositif d'oxymétrie de pouls avec mesure de PTT.
